# EUROPEAN PATENT APPLICATION

(11) **EP 4 797 540 A1**
(43) Date of publication of application: **26.08.2026**
(21) Application number: 25227300.8
(22) Date of filing: 26.12.2025
(51) Int. Cl.: H03M 7/30, G01N 33/487, A61B 5/24, G16H 50/20

(54) **DATA PROCESSING METHOD**

(30) Priority: 25.02.2025 JP 2025028047
(71) Applicant: SCREEN Holdings Co., Ltd., Kyoto-shi, Kyoto 602-8585 (JP)
(72) Inventor: JIKO, Takeshi, Osaka, 567-0085 (JP)
(74) Representative: Goddar, Heinz J.

(57) **Abstract**

With this data processing method, a thinning-out step and a compression step are performed on input data items (D1 to D16) to obtain compression data (C). The thinning-out step (S2) involves a peak-point extraction step (S202) of extracting only peak points in the input data items (D1 to D16). In the compression step (S3), the compression data (C) is generated by changing the data format of thinned-out data items (T1 to T16) generated across multiple channels in the thinning-out step (S2) such that the number of offset bytes for every one of all channels and a channel data item for every one of all the channels are arranged in orderly sequence. In each channel data item, the number of skipped points for every one of all data points in the channel data item and a difference in data value for every one of all the data points are arranged in orderly sequence. This thinning-out and compression of data enable reversibly and appropriately compressing the data while avoiding a loss of essential signal elements. That is, it is possible to appropriately compress digital data measured at multiple points across a plurality of channels by using a multi-electrode array device.

## Description

### BACKGROUND OF THE INVENTION

### Field of the Invention

The present invention relates to a data processing method for measurement data on a multi-electrode array.

### Description of the Background Art

In the field of electrophysiology, analyses are conducted on the activity of ion channels within a cell or a group of cells. Examples of known ion channel analyzers include intracellular action potential detectors using a patch-clamp technique and extracellular action potential detectors using multi-electrode array (MEA) devices. One example of the multi-electrode array devices is disclosed in Japanese Unexamined Patent Application Publication (Translation of PCT Application) No. 2023-160374.

Using the MEA devices, it is possible to simultaneously measure current values or voltage values of multiple electrodes. Using this feature enables simultaneous multipoint measurements of extracellular potentials arising from the activities of cells such as neurons or cardiocytes.

In recent years, advancements in device miniaturization technology have enabled the development of MEA devices with a higher number of electrodes than conventional MEAs or new MEA devices called CMOS-MEAs with 1000 units of ultra-fine electrodes. As the number of electrodes increases in this way, the volume of data becomes enormous when measurement data is obtained simultaneously across multiple channels. This creates the problem that enormous data capacity is required for data storage.

To address this problem, a data thinning-out method using a low-pass filter is generally used to reduce data size, but if data is thinned out until sufficiently compressed, essential signal elements may be lost. Additionally, there are also lossless compression methods such as difference methods in which data is compressed while leaving essential elements in the data, but such methods can only reduce data size by a maximum of about 50%. Therefore, new data compression methods specialized for electrical signals acquired by MEA devices are required.

### SUMMARY OF THE INVENTION

It is an object of the present invention to provide a technique capable of appropriately compressing digital data measured simultaneously at multiple points across multiple channels by using a multi-electrode array device.

To solve the problem described above, a first aspect of the present application is a data processing method for time-series digital data items measured simultaneously at multiple points across a plurality of channels, the time-series digital data items being measurement data on a multi-electrode array. The data processing method is executed by a computer to execute a) performing thinning-out processing on input data items obtained across all of the plurality of channels to generate thinned-out data items, b) changing a format of the thinned-out data items to generate compressed data, and c) storing the compressed data in a storage. The operation a) includes a1) extracting only a peak point in the time-series digital data items. In the operation b), in the compressed data, the number of offset bytes indicating the data starting point for every one of all of the plurality of channels and a channel data item for every one of all of the plurality of channels are stored in orderly sequence. In each channel data item, the number of points skipped from a previous data point for every one of all data points and a difference in data value from the previous data point for every one of all the data points are arranged in orderly sequence.

A second aspect of the present application is the data processing method according to the first aspect, in which the operation a) further includes a2) thinning out a location where a plurality of peak points, each being the peak point, appear within a predetermined period of time, in data generated in the operation a1).

A third aspect of the present application is the data processing method according to the second aspect, in which in the operation a2), when the peak point to be thinned out appears consecutively within a predetermined period of time, the peak point appearing consecutively is not thinned out.

A fourth aspect of the present application is the data processing method according to the third aspect, in which the operation a) further includes a3) thinning out the peak point whose absolute value is less than or equal to a predetermined threshold value, in data generated in the operation a2).

A fifth aspect of the present application is the data processing method according to the fourth aspect, in which the threshold value used in the operation a3) is a multiple of a constant of standard deviations of a predetermined number of initial data values in the time-series digital data items.

According to the first to fifth aspects of the present application, it is possible to appropriately compress digital data measured simultaneously at multiple points across multiple channels by using a multi-electrode array device, without a loss of essential signal elements.

In particular, according to the second aspect, it is possible to appropriately and further compress data.

In particular, according to the third aspect, it is possible to avoid a loss of essential signal elements in data.

In particular, according to the fourth and fifth aspects, it is possible to appropriately and further compress data.

These and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a diagram schematically showing configurations of a measurement apparatus and a data processing device according to one embodiment.
Fig. 2 is a functional block diagram of the data processing device according to one embodiment.
Fig. 3 is a flowchart showing a procedure for data processing according to one embodiment.
Fig. 4 is a flowchart showing a procedure for a thinning-out step according to one embodiment.
Fig. 5A is a diagram showing an example of first data items according to one embodiment.
Fig. 5B is a diagram showing an example of second data items according to one embodiment.
Fig. 5C is a diagram showing an example of thinned-out data items according to one embodiment.
Fig. 6A is a diagram showing an example of the first data items according to one embodiment.
Fig. 6B is a diagram showing an example of the second data items according to one embodiment.
Fig. 6C is a diagram showing an example of the thinned-out data items according to one embodiment.
Fig. 7 is a diagram showing one example of a data format of measurement data according to one embodiment.
Fig. 8 is a diagram showing one example of a data format of the first data items according to one embodiment.
Fig. 9 is a diagram showing one example of data formats of the second data items and the thinned-out data items according to one embodiment.
Fig. 10 is a diagram showing one example of a data format of compressed data according to one embodiment.
Fig. 11 is a functional block diagram of a data processing device according to a variation.
Fig. 12 is a flowchart showing a procedure for the thinning-out step according to the variation.

### DESCRIPTION OF THE PREFERRED EMBODIMENTS

Hereinafter, an embodiment of the present invention will be described with reference to the accompanying drawings. Note that constituent elements described in the embodiment are merely examples, and the scope of the present invention is not intended to be limited thereto. To facilitate understanding of the drawings, the dimensions or number of each constituent element may be illustrated in exaggerated or simplified form as necessary.

### 1. Measurement Apparatus Using MEA Device

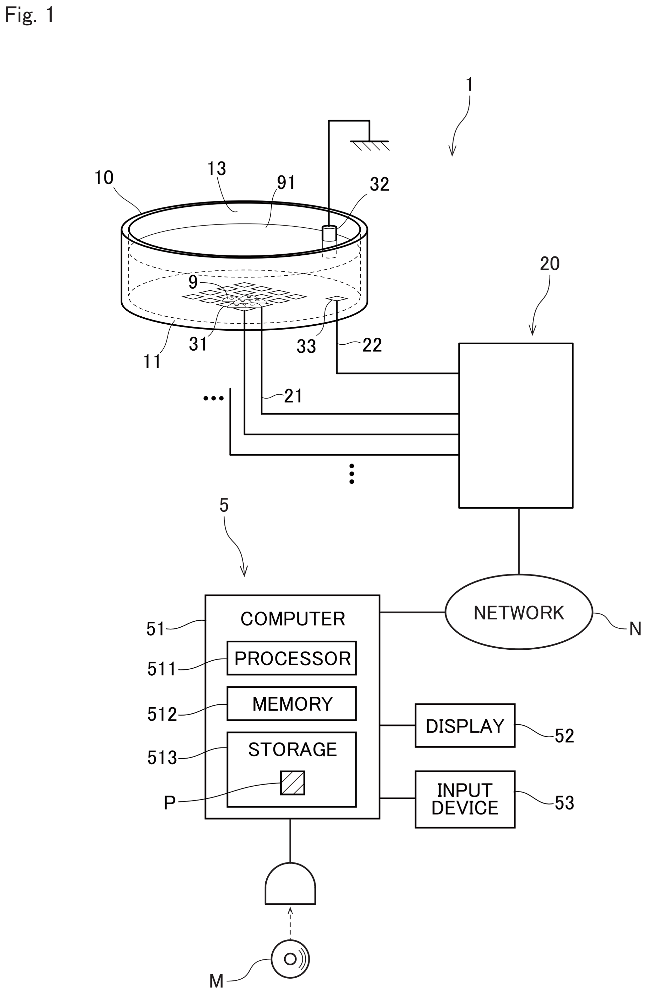

First, a configuration of a measurement apparatus 1 is described with reference to Fig. 1 as one example of a measurement device for acquiring time-series digital data that is measured simultaneously at multiple points and that is targeted for a data processing method according to an embodiment. Fig. 1 is a diagram schematically showing the configuration of the measurement apparatus 1. As shown in Fig. 1, the measurement apparatus 1 includes a measuring container 10 and a measurement unit 20.

The measuring container 10 is a container for measuring extracellular potentials, extracellular voltages, or impedances of cells 9 serving as target objects. The measuring container 10 contains a cell suspension dropped therein and including the cells 9. As shown in Fig. 1, the measuring container 10 has a bottom 11 and a side wall 13. The bottom 11 expands in a disc-like shape along a horizontal plane. The side wall 13 extends upward in a cylindrical shape from the peripheral edge of the bottom 11. The upper surface of the bottom 11 corresponds to the bottom surface inside the measuring container 10 in which the cell suspension is dropped. Note that the target to be measured by the measuring container 10 is not limited to the cells 9 and may, for example, be a sliced sample of a biomedical tissue.

The measuring container 10 includes a plurality of first electrodes 31, a second electrode 32, and a third electrode 33. The first electrodes 31 and the third electrode 33 are located at the upper surface of the bottom 11 (the bottom surface of the measuring container 10). Wiring lines that connects the first and third electrodes 31 and 33 and electrodes that can be connected to external devices are also located at the upper surface of the bottom 11 (the bottom surface of the measuring container 10). The wiring lines are covered with an insulator (e.g., light-sensitive polyimide). The first electrodes 31, the third electrode 33, and the aforementioned wiring lines are formed by, for example, photolithography.

The first electrodes 31 are arranged in an array. The measuring container 10 is a multi-electrode array (or a micro-electrode array) device in which a plurality of first microelectrodes 31 are arranged in an array. In the example shown in Fig. 1, 16 first electrodes 31 are arranged in a 4-by-4 matrix. Note that the number and arrangement of the first electrodes 31 can be set arbitrarily. Preferably, 10 or more first electrodes 31 may be arranged. The first electrodes 31 each have a square shape when viewed from above. Alternatively, the shape of the first electrodes 31 may be polygonal, circular, or any other shape other than square.

The second electrode 32 is located within the measuring container 10. For example, the second electrode 32 may be stick-shaped, and at least part of the second electrode 32 (e.g., the lower end) may be immersed in the measuring container 10.

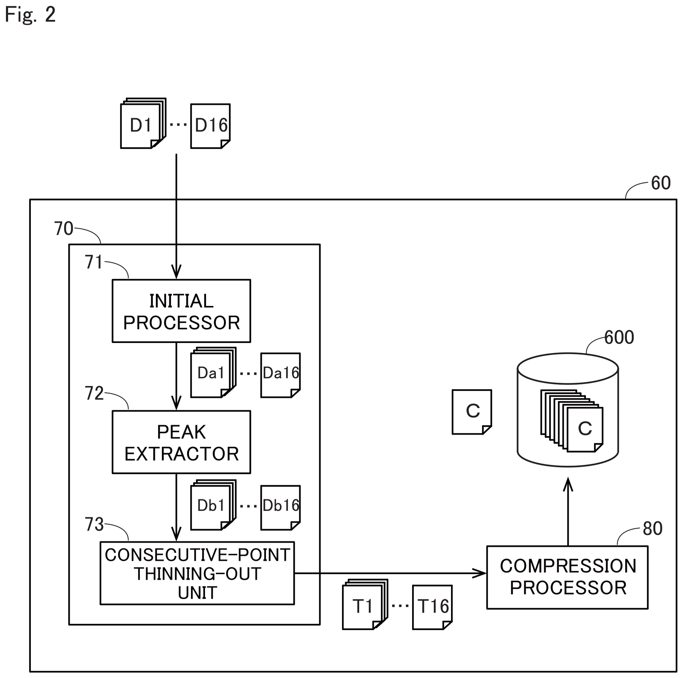

The third electrode 33 is located radially outward of and away from the first electrodes 31. The first electrodes 31 and the third electrode 33 are isolated from each other. In the examples shown in Figs. 1 and 2, the third electrode 33 has a square shape when viewed from above. Alternatively, the shape of the third electrode 33 may be polygonal, circular, or any other shape other than square.

The measurement unit 20 includes a plurality of first wiring lines 21 that are electrically connected to the plurality of first electrodes 31, respectively, and a second wiring line 22 that is electrically connected to the third electrode 33. From the first and second wiring lines 21 and 22, the measurement unit 20 can acquire electrical measurement values, such as the current values, voltage values, or impedances of the first electrodes 31, as time-series digital data. In the example shown in Fig. 1, 16 simultaneous measurement data items, i.e., input data items D1 to D16 that are times-series digital data, are acquired for 16 first electrodes 31.

### 2. Data Processing Device

Next, a data processing device 5 according to one embodiment is described with reference to Figs. 1 and 2. Fig. 2 is a functional block diagram of the data processing device 5.

As shown in Fig. 1, the data processing device 5 includes a computer mainframe 51, a display 52, and an input device 53. The computer mainframe 51 includes a processor 511 such as a CPU, memory 512 such as random access memory (RAM), and a storage 513 such as a hard disk drive. The display 52 displays graphs, images, and so on that are output from the computer mainframe 51. For example, the display 52 may be a liquid crystal display such as a personal computer (PC) monitor. The input device 53 is capable of receiving input of commands that are given to the computer mainframe 51. For example, the input device 53 may include a keyboard and a mouse. Note that the display 52 and the input device 53 may be configured as an integral unit such as a touch panel.

The memory 512 and the storage 513 are connected to the processor 511 via bus wiring (not shown). The storage 513 stores a computer program P. The processor 511 loads the computer program P stored in the storage 513 into the memory 512 and sequentially executes codes contained in the computer program P. In this way, the data processing device 5 performs data processing on a plurality of time-series digital data items that have been input.

The computer program P is application software for causing the computer mainframe 51 to execute various steps related to the aforementioned image processing. The computer program P is read from a storage medium M such as a CD or a DVD and installed into the computer mainframe 51. Alternatively, the computer program P may be downloaded into the computer mainframe 51 via a network N such as the Internet.

The input data items D1 to D16, which are a plurality of time-series digital data items measured by the measurement apparatus 1, are input to the data processing device 5. The data processing device 5 then performs compression processing on the input data items D1 to D16 to generate compressed data C.

As shown in Fig. 2, the computer mainframe 51 includes a data processor 60 as a processing unit implemented in software. The data processor 60 includes a thinning-out processor 70 and a compression processor 80.

The following description is given on the assumption that the input data items D1 to D16 obtained across 16 channels are each digital data consisting of 10,000 values acquired over a period of 500 ms at a sampling rate of 20,000 Hz. The number of channels, i.e., the number of data items that can be measured simultaneously, is not limited to 16, and may be any arbitrary number. The number of channels may, for example, be 384, 5000, or a larger value such as three millions.

The thinning-out processor 70 performs thinning-out processing on each of all the input data items D1 to D16 that have been input, to generate thinned-out data items T1 to T16 serving as processed data.

The thinning-out processor 70 includes an initial processor 71, a peak extractor 72, and a consecutive-point thinning-out unit 73. The initial processor 71 performs numbering processing on data points in each of the input data items D1 to D16 to generate first data items Da1 to Da16. The peak extractor 72 performs peak extraction processing for extracting only peak points on the first data items Da1 to Da16 to generate second data items Db1 to Db16. The consecutive-point thinning-out unit 73 performs consecutive-point thinning-out processing for thinning out consecutive peak points in the second data items Db1 to Db16 to generate thinned-out data items T1 to T16.

The compression processor 80 generates compressed data C by changing the data format of the thinned-out data items T1 to T16 and stores the compressed data C in a data storage 600. The data storage 600 may be the above-described storage 513, or may be a separately provided storage. The data storage 600 may also be an external storage medium connected to the computer mainframe 51.

Details of each processing performed by the thinning-out processor 70 and the compression processor 80 will be described later.

### 3. Procedure for Data Processing

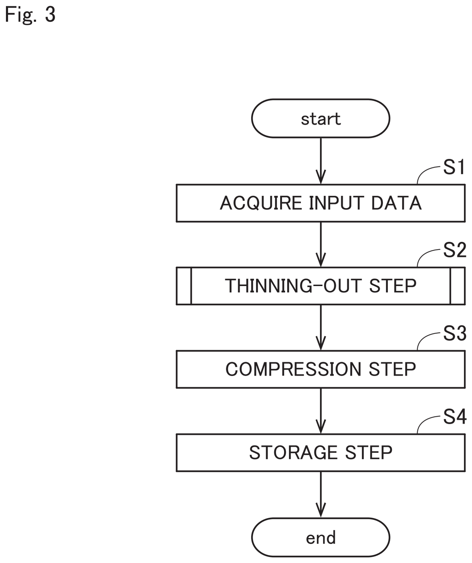

Next, a procedure for the data processing performed by the data processing device 5 is described with reference to Fig. 3. Fig. 3 is a flowchart showing the procedure for the data processing. The following description is given with reference to only the input data item D1 as a representative among the 16 input data items D1 to D16 on which the same processing is performed.

As shown in Fig. 3, the data processor 60 first acquires the input data items D1 to D16, which are time-series digital data, as input data to be processed (step S1). Specifically, the data processor 60 receives the input data items D1 to D16 from the measurement apparatus 1. At this time, the acquired input data items D1 to D16 may be once stored in the data storage 600, or may be subjected directly to data processing performed by the thinning-out processor 70.

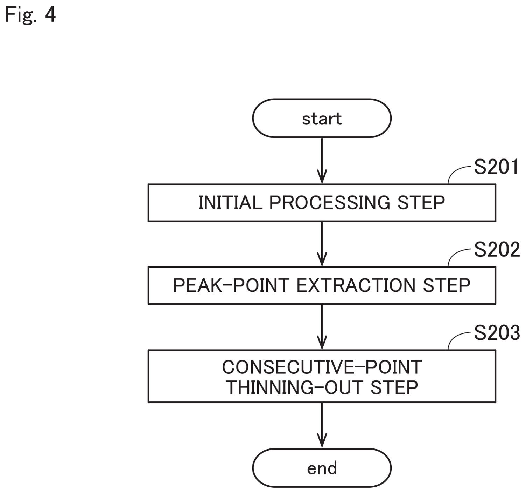

Then, the thinning-out processor 70 performs thinning-out processing on each of the input data items D1 to D16 (step S2: thinning-out step). Fig. 4 is a flowchart showing a detailed procedure for the thinning-out step S2. In the thinning-out step S2, first as shown in Fig. 4, the initial processor 71 of the thinning-out processor 70 performs initial processing on each of the input data items D1 to D16 to generate the first data items Da1 to Da16 (step S201: initial processing step).

Specifically, in the case where the input data item D1 is data in which only measurement data items representing measurement values are arranged in time sequence without being assigned times or values corresponding to times, the initial processor 71 first assigns numbers 1, 2, 3, ..., and 10,000 corresponding to times to the respective measurement values. Thus, in the case where each of the input data items D1 to D16 is digital data consisting of 10,000 values, the first data item Da1 after numbering consists of 2 × 10,000 values. Hereinafter, the newly assigned numbering value is referred to as the "number", the original value of the input data item D1 is referred to as the "data value", and the corresponding number and data value are collectively referred to as the "data set".

Then, the peak extractor 72 performs peak-point extraction processing on each of the first data items Da1 to Da16 to generate the second data items Db1 and Db16 (step S202: peak-point extraction step).

Specifically, only data sets whose data values are peak values are extracted from the first data item Da1 to obtain the second data item Db1. Note that "being a peak value" means that data values before and after a data value concerned are both either smaller or larger than the data value concerned.

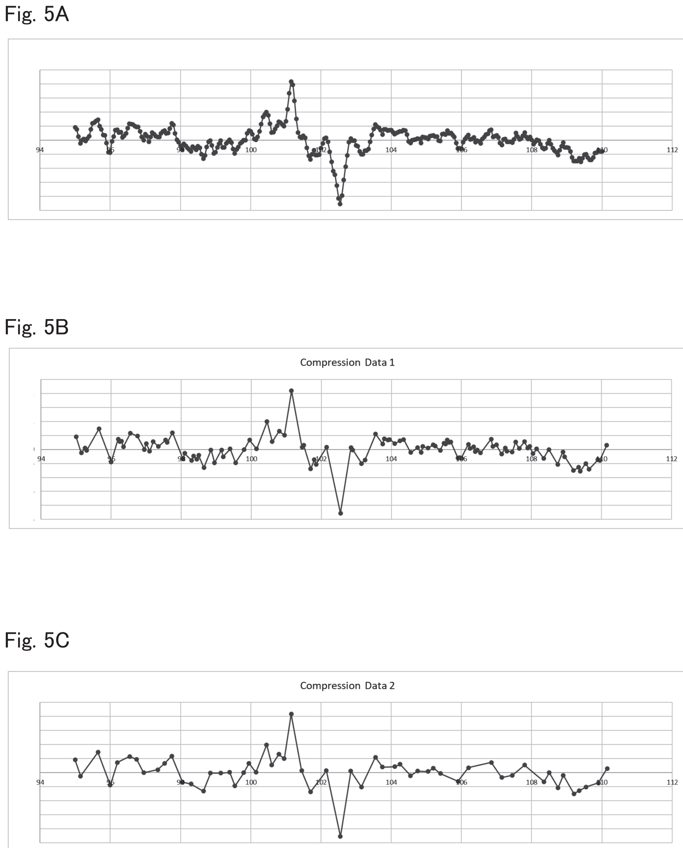

Figs. 5A to 5C are diagrams showing examples of the first data item Da1, the second data item Db1, and the thinned-out data T1. Fig. 5A shows an example of the first data item Da1, Fig. 5B shows an example of the second data item Db1, and Fig. 5C shows an example of the thinned-out data T1. In any of Figs. 5A to 5C, the horizontal axis indicates the number, and the vertical axis indicates the data value. Then, each data point configuring one data set is indicated by a single point (circular marker). A comparison between the first data item Da1 shown in Fig. 5A and the second data item Db1 shown in Fig. 5B shows that the second data item Db1 includes a smaller number of data points than the first data item Da1, but retains waveform characteristics of the first data item Da1.

Then, the consecutive-point thinning-out unit 73 thins out locations where a plurality of peak points appear within a predetermined period of time, in the second data items Db1 to Db16 to generate the thinned-out data items T1 to T16 (step S203: consecutive-point thinning-out step).

Specifically, the consecutive-point thinning-out unit 73 first determines locations where peak points appear consecutively within a short period of time, as consecutive points and extracts the consecutive points from the second data item Db1. That is, locations where a plurality of peak points appear within a predetermined period of time are extracted as consecutive points. For example, in the case where the predetermined period of time is set to two sampling periods, locations where the next peak point appears within two sampling periods after a given peak point, i.e., locations where the difference between the numbers of two adjacent peak points is within two, are extracted as consecutive points.

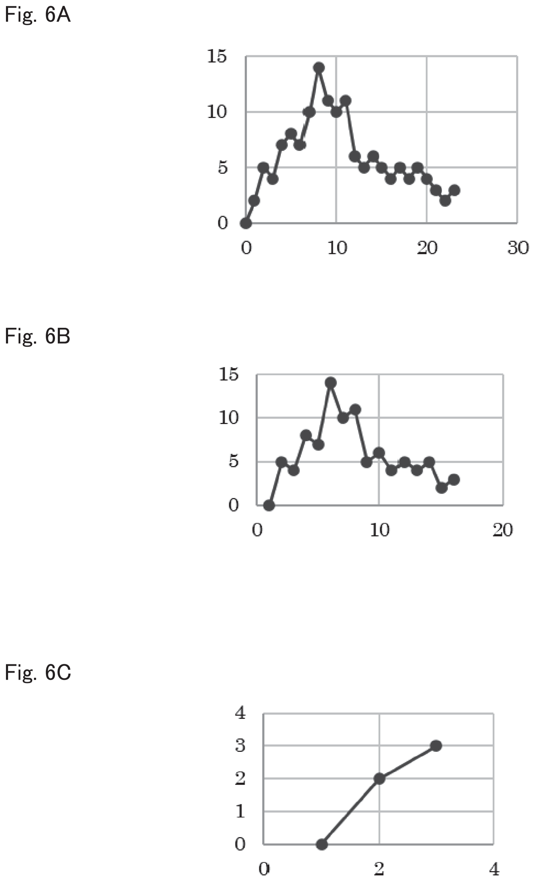

Figs. 6A to 6C are diagrams showing examples of the first data item Da1, the second data item Db1, and data obtained by thinning out all consecutive points in the second data item Db1 when the consecutive points appear in the second data item Db1. Fig. 6A shows an example of the first data item Da1, Fig. 6B shows an example of the second data item Db1, and Fig. 6C shows an example of the data obtained by thinning out all consecutive points in the second data item Db1. In any of Figs. 6A to 6C, the horizontal axis indicates the number, and the vertical axis indicates the data value.

As shown in Figs. 6A to 6C, a comparison between the first data item Da1 shown in Fig. 6A and the second data item Db1 shown in Fig. 6B shows that, in the examples shown in Figs. 6A to 6C, the second data item Db1 generated by extracting peak points from the first data item Da1 retains waveform characteristics of the first data item Da1. However, a comparison between the data items shown in Figs. 6A and 6B and the data shown in Fig. 6C shows that the data shown in Fig. 6C, obtained by thinning out all the consecutive points in the second data item Db1, fails to retain the waveform characteristics of the first data item Da1.

In this way, simply thinning out consecutive points may result in a loss of characteristic peak points when consecutive points appear consecutively due to influences such as noise.

In view of this, if a predetermined number (e.g., three) or more of consecutive points appear consecutively in the consecutive-point thinning-out step S203, the consecutive-point thinning-out unit 73 does not thin out the consecutive points. On the other hand, in the case where a predetermined number or more of consecutive points do not appear consecutively, the consecutive-point thinning-out unit 73 thins out the consecutive points. In this way, the thinned-out data items T1 to T16 are generated.

When the thinning-out step S2 has finished, then, the compression processor 80 performs compression processing on the thinned-out data items T1 to T16 to compress all the thinned-out data items T1 to T16 into one compressed data C (step S3: compression step). Then, the compression processor 80 stores the compressed data C in the data storage 600 (step S4: storage step).

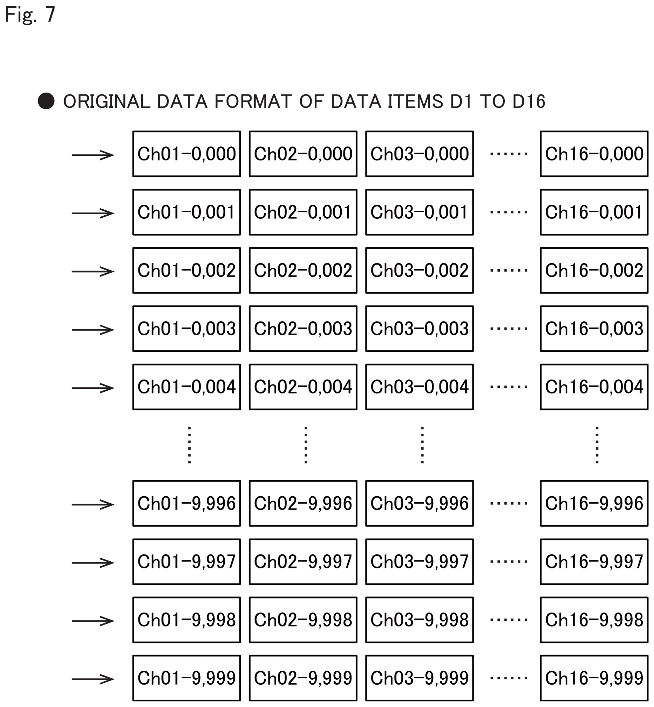

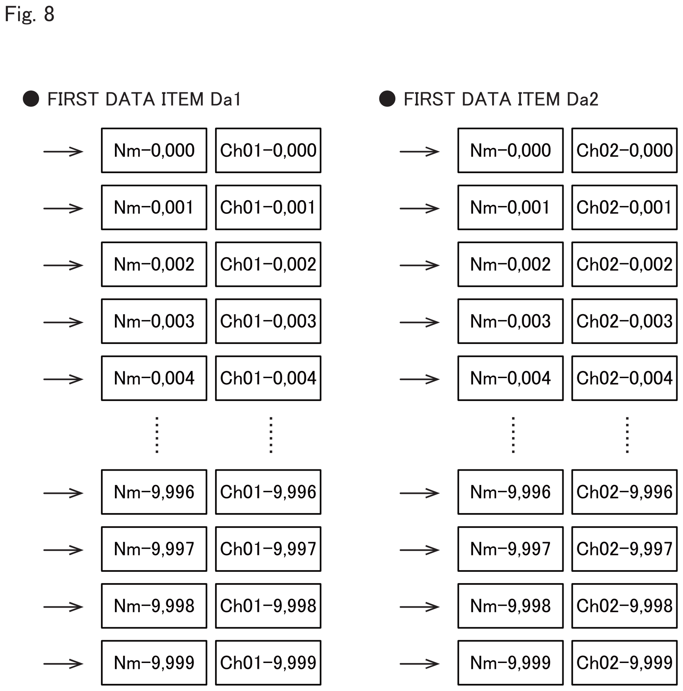

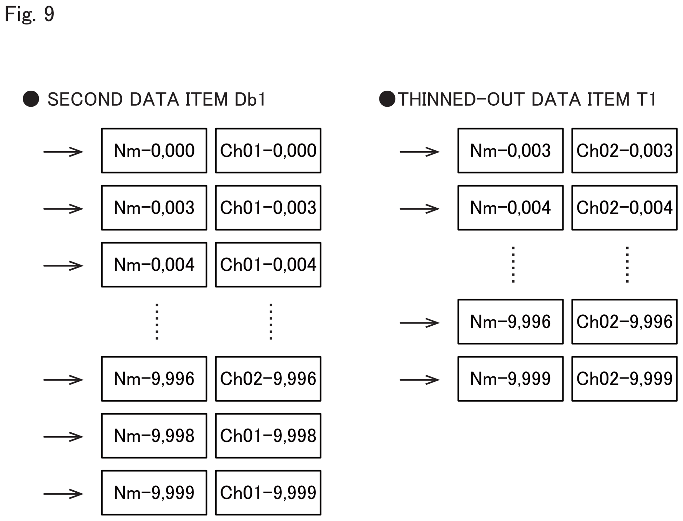

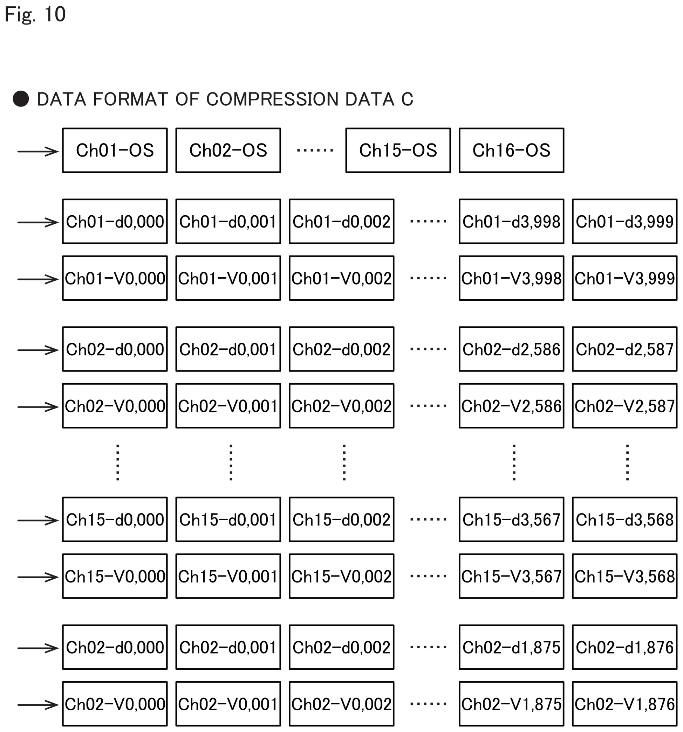

Figs. 7 to 10 shows examples of data formats of the respective data items. Fig. 7 shows an example of the data format of the input data items D1 to D16, which are raw measurement data. Fig. 8 shows an example of the data format of the first data items Da1 and Da2. Fig. 9 shows an example of the data formats of the second data item Db1 and the thinned-out data T1. Fig. 10 shows an example of the data format of the compressed data C. In the examples shown in Figs. 7 to 10, characters enclosed in squares that make up the matrix correspond to data fragments, and an arrow indicating the order of data is placed at the beginning of each row. The data shown in each of the examples in Figs. 7 to 10 is a set of data in which data fragments are stored sequentially from the top row to the bottom row, following the direction of the arrows.

In Figs. 7 to 9, each data fragment stored in a format of Ch[XX]-[Y,YYY] is a data value acquired at the time [Y,YYY] for the [XX]-th channel (the time is not the actual time but is the "number" indicating the order of data). Each data fragment stored in a format of Nm-[Y,YYY] is the value of the "number" itself indicating the time [Y,YYY].

The input data items D1 to D16, which are the measurement data, are acquired simultaneously for 16 channels at each sampling time. Thus, as shown in Fig. 7, the data to be stored first corresponds to 16 data fragments in the first row in Fig. 7, namely, the measurement value Ch01-0,000 for the first channel, the measurement value Ch02-0,000 for the second channel, the measurement value Ch03-0,000 for the third channel, ...(omitted)..., and the measurement value Ch16-0,000 for the sixteenth channel at the time 0,000. Following this, other 16 data fragments, i.e., the measurement values Ch01-0,001, Ch02-0,001, ...(omitted)..., and Ch16-0,001 for the 16 channels at the sampling time 0,001, are stored. In this way, the input data items D1 to D16 are acquired as a data block that includes the same number of sets of data fragments as the number of samplings, each set consisting of 16 data fragments.

In the initial processing step S201, as shown in Fig. 8, the data block of the input data items D1 to D16 is divided into the 16 first data items Da1 to Da16, and each measurement value is numbered. In the example shown in Fig. 8, the data sets, each consisting of the number and the data value, are stored in orderly sequence. Specifically, the first two data items stored in the first row of the first data item Da1 for the first channel are the number Nm-0,000 at the time 0,000 and the measurement value Ch01-0,000 at the time 0,000 for the first channel. The next two data items are the number Nm-0,001 at the time 0,001 and the measurement value Ch01-0,001 at the time 0,001 for the first channel, which are stored in the second row. The same also applies to the first data item Da2 for the second channel.

In the peak-point extraction step S202, points other than the extracted peak points are deleted, so that data sets indicated by rows in Fig. 8 are deleted. Accordingly, as shown in Fig. 9, the number of rows in the data (the length of columns) shown in Fig. 8 is reduced. Specifically in the example shown in Fig. 9, in the second data item Db1, the data sets at times 0,001, 0,002, and 9,997 are deleted as compared to the first data item Da1.

Moreover, in the example shown in Fig. 9, in the thinned-out data T1 in which more data points have been deleted in the consecutive-point thinning-out step S203, the data sets at times 0,000 and 9,998 are deleted as compared to the second data item Db1.

In the examples shown in Figs. 8 and 9, the data sets in the first data items Da1 to Da16, the second data items Db1 to Db16, and the thinned-out data items T1 to T16 are stored sequentially row by row from top to bottom, and within each row from left to right. That is, the data is stored in the order of number, data value, number, data value, and so on. Alternatively, the data may also be stored in orderly sequence, first from top to bottom in the left column and then from top to bottom in the right column, i.e., by first storing all of the numbers in orderly sequence and then storing all of the data values in orderly sequence.

In the case where the thinned-out data items T1 to T16 obtained in this manner are compressed into the compressed data C, the present embodiment employs the data format shown in Fig. 10.

In Fig. 10, data fragments stored in the format of Ch[XX]-OS are so-called offset bytes that express, in bytes, the distance from a data starting point of the data for the first channel to a data starting point of the data for the [XX]-th channel. In the example shown in Fig. 10, the data for the first channel starts from the beginning of the second row. This position is set as zero. Thus, the value of Ch01-OS is zero. The data for the second channel starts from the beginning of the fourth row. The value of Ch02-OS indicating this position represents the number of bytes used for the data in the second and third rows for the first channel. Similarly, the value of Ch03-OS represents a total number of bytes used for the data in the second and third rows for the first channel and bytes used for the data in the fourth and fifth rows for the second channel.

In Fig. 10, there are also data fragments stored in the formats of Ch[XX]-d[Z,ZZZ] and Ch[XX]-V[Z,ZZZ], where [XX] denotes the [XX]-th channel similarly to the above, and [Z,ZZZ] denotes the number indicating the order of the thinned-out data items T1 to T16. That is, in the thinned-out data item T1 shown in Fig. 9, data at the time 0,003 corresponds to [Z,ZZZ] = 0th, and data at the time 0,004 corresponds to [Z,ZZZ] = 1st.

The format of Ch[XX]-d[Z,ZZZ] indicates the number of data points skipped from the previous data point. That is, it indicates the position of the time in that data relative to the time in the previous data. Note that the first data point indicates the difference from the time 0,000. In the example of the thinned-out data item T1 shown in Fig. 9, since the first data point is acquired at the time 0,003, the value of Ch01-d0,000 is "0,003". Since the second data point is acquired at the time 0,004, the value of Ch01-d0,001 is a difference "0,001" from the time 0,003 of the first data point.

The format of Ch[XX]-V[Z,ZZZ] indicates the difference in data value from the previous data point. For the first data point, it is the data value itself. In the example of the thinned-out data item T1 shown in Fig. 9, Ch01-V0,000 corresponds to the data value itself of Ch01-0,003. Ch02-V0,001 corresponds to a difference value obtained by subtracting the data value of Ch01-0,003 from the data value of Ch01-0,004.

In this way, in the compressed data C shown in Fig. 10, the number of offset bytes indicating the data starting point for every one of all the channels and a channel data item for every one of all the channels are stored in orderly sequence. That is, in the compressed data, firstly the number of offset bytes for all of the 16 channels, namely, the number of offset bytes for the first channel, the number of offset bytes for the second channel, ...(omitted)..., and the number of offset bytes for the sixteenth channel, are stored in orderly sequence, and then the channel data items for all of the 16 channels, namely, the channel data item for the first channel, the channel data item for the second channel, ...(omitted)..., and the channel data item for the sixteenth channel, are stored in orderly sequence.

In the channel data item for each channel, the number of data points skipped from the previous data point for every one of all the data points and a difference in data value from the previous data point for every one of all the data points are stored in orderly sequence. Specifically, in the first channel data item, firstly the start position of the first data point in the thinned-out data item T1, the number of skipped data points for the second data point, the number of skipped data points for the third data point, ...(omitted)..., and the number of skipped data points for the last data point are stored in orderly sequence, and then the data value of the first data point in the thinned-out data item T1, the difference in data value from the second data point, the difference in data value from the third data point, ...(omitted)..., and the difference in data value from the last data point are stored in orderly sequence.

By creating the compressed data C in this format, it is possible to considerably compress data capacity. For example, in the case of the measurement data including the input data items D1 to D6 shown in Fig. 7, the capacity of one data fragment Ch[XX]-[Y,YYY] is two bytes. In this case, the data capacity of this measurement data is 2 × 16 × 10,000 = 320,000 bytes.

In contrast, in the compressed data C, the offset byte Ch[XX]-OS can be 4 bytes, the number of skipped points Ch[XX]-d[Z,ZZZ] can be 0.5 bytes (i.e., two in a pair makes one byte), and the difference in data value Ch[XX]-V[Z,ZZZ] can be 1 byte. Note that some differences in data value Ch[XX]-V[Z,ZZZ] may become 2 bytes depending on the data waveform, but there are only a small number of such differences.

If all of the differences in data value Ch[XX] and V[Z,ZZZ] are 2 bytes, and an average of the numbers of data points in the thinned-out data items T1 to T16 is 4,000, which is 40% of the number of data points in the original input data items D1 to D16, the data capacity of the compressed data C is 4 × 16 + (0.5 + 2) × 4,000 × 16 = 10,064 bytes. That is, the data capacity of the compressed data C in this case can be compressed to approximately 32% of the data capacity of the original input data items D1 to D16.

In the above description, the number of channels is 16 and the number of samples is 10,000, but the number of channels and the number of samples may be set arbitrarily.

By extracting peak points and deleting consecutive peak points in this manner, it is possible to appropriately compress the digital data of electrical signals measured using a multi-electrode array device while avoiding a loss of essential signal elements.

Moreover, by configuring the compressed data C in the above-described data format, it is possible to considerably and reversibly compress the digital data measured simultaneously at multiple points across multiple channels by using the multi-electrode array device.

### 4. Variations

While one embodiment has been described thus far, the present invention is not limited to the above-described embodiment.

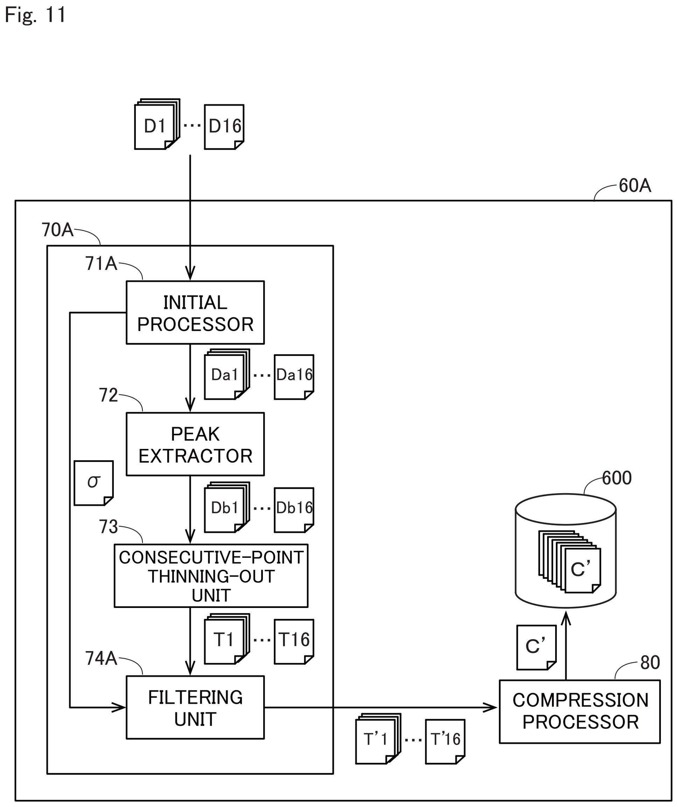

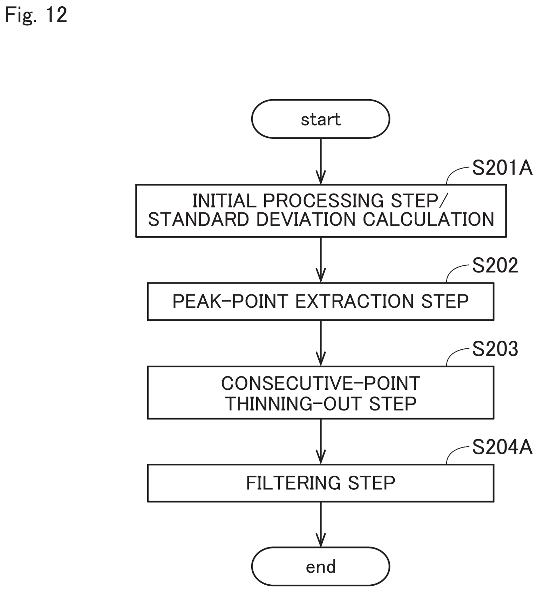

Fig. 11 is a functional block diagram of a data processor 60A according to a variation. Fig. 12 is a flowchart showing a procedure for a thinning-out step performed by the data processor 60A.

As shown in Fig. 11, in the data processor 60A, a thinning-out processor 70A further includes a filtering unit 74A. In the thinning-out step performed by the data processor 60A, as shown in Fig. 12, an initial processor 71A first generates first data items Da1 to Da16 and calculates standard deviations σ1 to σ16 of input data items D1 to D16, respectively (step S201A: initial processing step).

When calculating the standard deviation σ1 of the input data item D1, the initial processor 71A calculates, as the standard deviation σ1, a standard deviation of, not all of, but a predetermined number n of data values of the input data item D1 from the first data value to the n-th data value. The predetermined number n is a natural number determined in advance and may, for example, be 500.

Next, in the same manner as in the above-described embodiment, the peak extraction unit 72 performs peak point extraction processing on each of the first data items Da1 to Da16 to generate second data items Db1 to Db2 (step S202: peak-point extraction step).

Then, in the same manner as in the above-described embodiment, the consecutive-point thinning-out unit 73 thins out locations where a plurality of peak points appear within a predetermined period of time in each of the second data items Db1 to Db16 to generate thinned-out data items T1 to T16 (step S203: consecutive-point thinning step).

Thereafter, the filtering unit 74A performs filtering processing on the thinned-out data items T1 to T16 to generate second thinned-out data items T'1 to T'16 (step S204A: filtering step).

Specifically, the filtering unit 74A calculates threshold values α*σ1 to α*σ16 by multiplying the calculated standard deviations σ1 to σ16 by a constant α. The constant α is a predetermined positive value and may, for example, be 1. Alternatively, the constant α may be a value less than 1 such as 0.3 or 0.7, or may be a value greater than 1 such as 1.2 or 1.6, depending on the waveform characteristics of the acquired input data items D1 to D16.

Then, the filtering unit 74A thins out data points whose absolute data values are smaller than the threshold values α*σ1 to α*σ16 in the data points of the thinned-out data items T1 to T16 to generate the second thinned-out data items T'1 to T'16. Thereafter, the compression processor 80 compresses the second thinned-out data items T'1 to T'16 to generate compressed data C'. At this time, the values of the standard deviations σ1 to σ16 may be stored at the end of the compressed data C' and used for subsequent data analysis.

In this way, in the examples shown in Figs. 11 and 12, values near zero that are less likely to be characteristic are thinned out in the analysis of electrical signals measured using a multi-electrode array device. Accordingly, it is possible to appropriately reduce data capacity while retaining characteristic points.

While the invention has been shown and described in detail, the foregoing description is in all aspects illustrative and not restrictive. It is therefore understood that numerous modifications and variations that are not described above can be devised without departing from the scope of the invention. The configurations in the embodiment and variations described above may be appropriately combined or omitted as long as there are no mutual inconsistencies.

## Claims

1. A data processing method for time-series digital data items (D1 to D16) measured simultaneously at multiple points across a plurality of channels, the time-series digital data items being measurement data on a multi-electrode array,
the data processing method being executed by a computer (51) to execute:
a) thinning-out step (S2) of performing thinning-out processing on input data items (D1 to D16) obtained across all of the plurality of channels to generate thinned-out data items (T1 to T16);
b) compression step (S3) of changing a format of the thinned-out data items (T1 to T16) to generate compression data (C); and
c) storage step (S4) of storing the compression data (C) in a storage (600),
wherein the operation a) includes:
a1) peak-point extraction step (S202) of extracting only a peak point in the time-series digital data items (D1 to D16),
in the operation b), in the compression data (C), the number of offset bytes indicating the data starting point for every one of all of the plurality of channels and a channel data item for every one of all of the plurality of channels are stored in orderly sequence, and
in each channel data item, the number of points skipped from a previous data point for every one of all data points and a difference in data value from the previous data point for every one of all the data points are arranged in orderly sequence.

2. The data processing method according to claim 1, wherein
the operation a) further includes:
a2) consecutive-point thinning-out step (S203) of thinning out a location where a plurality of peak points, each being the peak point, appear within a predetermined period of time, in data generated in the operation a1).

3. The data processing method according to claim 2, wherein
in the operation a2), when the peak point to be thinned out appears consecutively within a predetermined period of time, the peak point appearing consecutively is not thinned out.

4. The data processing method according to claim 3, wherein
the operation a) further includes:
a3) filtering step (S204A) of thinning out the peak point whose absolute value is less than or equal to a predetermined threshold value, in data generated in the operation a2).

5. The data processing method according to claim 4, wherein
the threshold value used in the operation a3) is a multiple of a constant of standard deviations of a predetermined number of initial data values in the time-series digital data items.
